# EUROPEAN PATENT SPECIFICATION

(11) **EP 3 806 795 B1**
(45) Date of publication and mention of the grant of the patent: **25.10.2023**
(21) Application number: 19734612.5
(22) Date of filing: 13.06.2019
(51) Int. Cl.: A61F 13/471, A61F 13/491

(54) **ABSORBENT ARTICLE HAVING RECESSED ZONE**
SAUGFÄHIGER ARTIKEL MIT VERTIEFTEN ZONEN
ARTICLE ABSORBANT AYANT UNE ZONE ÉVIDÉE

(30) Priority: 13.06.2018 US 201862684255 P
(43) Date of publication of application: 21.04.2021
(73) Proprietor: Attends Healthcare Products, Inc., Raleigh, NC 27617 (US)
(72) Inventor: OTTERY, Trent, Raleigh, NC 27617 (US); VROOMAN, Jacob, Raleigh, NC 27617 (US)
(74) Representative: Lohr, Jöstingmeier & Partner
(86) International application number: PCT/US2019/036887
(87) International publication number: WO 2019/241464

(56) References cited:
- EP-A1- 1 614 406
- WO-A1-99/33422
- WO-A1-2017/058069
- FR-A1- 2 701 389
- US-A- 5 651 778
- US-A1- 2012 245 547
- US-A1- 2017 079 858

## Description

### CROSS-REFERENCE TO RELATED APPLICATIONS

This application claims priority to and the benefit of U.S. Provisional Application No. 62/684,255, filed June 13, 2018.

### FIELD OF INVENTION

The present invention relates generally to absorbent products like incontinence guards, adult incontinence briefs, protective underwear, infant diapers, training pants, and the like; and more particularly, but not by way of limitation, to absorbent products configured to accommodate male anatomy.

### BACKGROUND

Examples of disposable absorbent articles that are wearable by a user include baby diapers, training pants, adult incontinence briefs and underwear, and bladder control pads, all of which may be made in disposable forms. "Disposable" refers to articles that are designed to be discarded after a limited use rather than being laundered or otherwise restored for reuse. Disposable absorbent products have met with widespread acceptance in the marketplace for a variety of applications, including infant and adult incontinence care, in view of the manner in which such products can provide effective and convenient liquid absorption and retention while maintaining the comfort of the wearer. Such disposable absorbent articles often include a topsheet that is configured to be closest to the wearer during use, a liquid-impermeable backsheet or outer cover, and an absorbent core between the topsheet and the backsheet. In some instances, such disposable articles also include an acquisition-distribution layer (ADL) disposed between the topsheet and the absorbent core.

Such disposable absorbent articles also include, for example, pads and incontinence guards for light to moderate incontinence uses, i.e., pads and guards adapted to absorb urine. Many men suffer from light to moderate urinary incontinence. Most protective, incontinence guards available on the market today for male bladder control issues are pads designed for women. Examples of such pads or guards are disclosed in U.S. Patent No. 4,804,380 and U.S. Patent No. 5,423,787. While some pads are made wider for male use, these pads are not designed to address the significant anatomical differences between men and women and thus can cause discomfort and are susceptible to leakage when used by men. Accordingly, there is a need in the art for absorbent articles that accommodate the male anatomy to increase comfort, improve anatomic stabilization, and manage leakage for male wearers.

WO 99/33422 A discloses articles adapted for the adult male anatomy. The articles include a moisture barrier, a liner bonded to the moisture barrier, and an absorbent assembly sandwiched therebetween. The moisture barrier is gathered along each of its side edges between forward and rearward terminal points. The moisture barrier is gathered along its back end by an elasticized back pouch member. The back pouch member includes a flange section and a pouch section. The pouch section includes a substantially fixed edge portion, an elasticized gathered movable edge portion, a substantially liquid impermeable pouch barrier layer, a pouch fabric layer, and a plurality of separate laterally extending pouch elastic members sandwiched between the pouch barrier layer and the pouch fabric layer.

EP1614406A1 suggests an absorbent pad for men. The absorbent pad comprises a liquid permeable top sheet, a back sheet and an absorbent member between the top sheet and the back sheet. A pair of liquid impermeable gather sheets face each other on the top sheet. A liquid permeable auxiliary sheet is provided to extend from a side of one of said gather sheets toward the other one of said gather sheets between said top sheet and said gather sheets, and an opening for inserting male genitals is formed by the auxiliary sheet and said other gather sheet.

FR2701389 A1 relates to an absorbent article for males. The article has waterproof layers, integral in a sealed manner with their edges, with the exception of one end to thereby to provide, on the one hand, a pocket for receiving urine and, on the other hand, an opening allowing the introduction of the phallus in the pocket.

US 2012 0245547 A1 discloses an incontinence garment with a front portion and a rear portion. The front portion has a body with a shell forming a cavity adapted to accommodate male genitalia. An absorbent body is disposed within the cavity and a body liquid-permeable liner is attached to the shell.

US 5,651,778 A suggest to manufacture an incontinence article by providing a formable, liquid impermeable moisture barrier material, an absorbent assembly, and a liquid permeable liner material. The moisture barrier and a composite structure including the moisture barrier is heated to the softening point of the moisture barrier. By vacuum forming to structure is conformed to the shape of a mold surface.

WO 2017/058069 A1 relates to a disposable pant-type absorbent article. The article has a chassis, an absorbent core, a front region configured for receiving the genitals of a male user. The front section is made of an elastic material. A first portion of the elastic material extends over at least a central portion of the absorbent article for pressing a front portion of the absorbent core towards a user during use of the absorbent article. A second portion of the elastic material is located more towards a back and has an a side elastic element extending along the transverse sides of the front region. The absorbent article further comprises an elongated side elastic element attached in a substantially longitudinal direction on each transverse side of the front region for providing a side gathering effect along the transverse sides of the front region, such that the absorbent core within the front region, under the influence of the elongated side elastic elements and the first portion of the elastic material bulges outwardly during use of the article to form a bowl shaped portion for receiving the genitals of a male user.

### SUMMARY

The present absorbent articles address the above-noted limitations of conventional absorbent articles by providing a chassis that has a variable thickness to define a recess. Such a recess can receive a wearer's phallus and thereby improve anatomic stabilization and decrease the pressure exerted on the phallus to increase comfort for male wearers. Accommodation of the phallus within a recess can also prevent the phallus from urging the present absorbent articles away from the wearer during use. Additionally, recesses of the present absorbent articles can facilitate containment of liquids received therein. The present absorbent articles thereby have improved leakage management compared to conventional absorbent articles.

Some of the present articles comprise a chassis having a length extending longitudinally between opposing front and rear edge portions. Some articles have a recessed region disposed between first and second longitudinally edge portions. In some articles, the thickness of the chassis within the recessed region is at least 10% smaller than a thickness of the chassis within each of the first and second longitudinal edge portions such that the chassis defines a longitudinally-extending recess.

In some articles, the recess is configured to receive a phallus of a wearer. The recess, in some articles, extends along a longitudinal distance that is at least one-third of the length of the chassis and, optionally, is at least 10% shorter than the length of the chassis. The recess, in some articles, is disposed closer to the front edge portion than to the rear edge portion and, optionally, extends from the front edge portion. In some articles, a length of the recess, measured along a longitudinal direction, is at least 5 cm. In some articles, a width of the recess, measured along a latitudinal direction extending between the first and second longitudinal edges, is at least 3 cm. In some articles, a depth of the recess, measured along a direction perpendicular to the latitudinal and longitudinal directions, is at least 1.5 cm.

Some articles have a retaining member that extends between the first and second longitudinal edge portions over at least a portion of the recess such that the retaining member and the recess together define a chamber and an opening. In some articles, the opening is configured to receive a phallus of a wearer into the chamber and, optionally, is disposed closer to the front edge portion than is the retaining member.

In some articles, the chassis has absorbent material within the recessed region and, optionally, within each of the edge portions. In some articles, the absorbent material comprises a laminate. The laminate, in some articles, has an inner lamina disposed between first and second outer laminae. In some articles, the inner lamina comprises superabsorbent polymer (SAP). The first outer lamina, in some articles, comprises tissue, and the second outer lamina, in some articles, comprises at least one of a tissue and a nonwoven, optionally a carded nonwoven. The absorbent material, in some articles, comprises a core having fluff and SAP. In some articles, the core is disposed on the laminate such that, when the absorbent article is worn by a wearer, the core is disposed closer to the wearer than is the laminate. In some of such articles, the absorbent material comprises a through-air bonded polymer nonwoven disposed between the laminate and the core.

In some articles, the laminate is folded such that the chassis comprises one or more folded layers of the laminate within the recessed region and, for each of the first and second longitudinal edge portions, two or more folded layers of the laminate such that there are more folded layers within the longitudinal edge portion than within the recessed region.

Some articles comprise an incontinence guard. In some of such articles, the chassis has opposing inner and outer surfaces and an adhesive disposed on the outer surface, wherein, when the article is worn by a wearer, the inner surface faces the wearer and the adhesive is configured to adhere to a clothing article of the wearer. In some articles, a first side panel of the front edge portion is configured to be coupled to a first side panel of the rear edge portion and a second side panel of the front edge portion is configured to be coupled to a second side panel of the rear edge portion to define a closed configuration in which the front and rear edge portions cooperate to encircle and define a waist opening, a left side of the chassis defines a first leg opening, and a right side of the chassis defines a second leg opening.

Some of the present methods of making an absorbent article comprise folding a laminate to form at least a part of a chassis. In some methods, folding is performed such that the chassis has two or more folded layers of the laminate within each of a first longitudinal edge portion and a second longitudinal edge portion and one or more folded layers of the laminate within a recessed region disposed between the first and second longitudinal edge portions. In some of such methods, folding is performed such that each of the first and second longitudinal edge portions has more folded layers than the recessed region such that a thickness of the chassis within the recessed region is at least 10% smaller than a thickness of the chassis within each of the first and second longitudinal edge portions, the chassis thereby defining a longitudinally-extending recess configured to receive a phallus of a wearer. In some of such methods, the laminate has an inner lamina disposed between first and second outer laminae, the inner lamina comprising superabsorbent polymer (SAP), the first outer lamina comprising tissue, and the second outer lamina comprising at least one of a tissue and a nonwoven, optionally a carded nonwoven.

The term "coupled" is defined as connected, although not necessarily directly, and not necessarily mechanically; two items that are "coupled" may be unitary with each other. The terms "a" and "an" are defined as one or more unless this disclosure explicitly requires otherwise. The term "substantially" is defined as largely but not necessarily wholly what is specified - and includes what is specified; e.g., substantially 90 degrees includes 90 degrees and substantially parallel includes parallel - as understood by a person of ordinary skill in the art. In any disclosed embodiment, the term "substantially" may be substituted with "within [a percentage] of" what is specified, where the percentage includes 0.1, 1, 5, and 10 percent.

The terms "comprise" and any form thereof such as "comprises" and "comprising," "have" and any form thereof such as "has" and "having," and "include" and any form thereof such as "includes" and "including" are open-ended linking verbs. As a result, an apparatus that "comprises," "has," or "includes" one or more elements possesses those one or more elements, but is not limited to possessing only those elements. Likewise, a method that "comprises," "has," or "includes" one or more steps possesses those one or more steps, but is not limited to possessing only those one or more steps.

Any embodiment of any of the apparatuses, systems, and methods can consist of or consist essentially of - rather than comprise/include/have - any of the described steps, elements, and/or features. Thus, in any of the claims, the term "consisting of" or "consisting essentially of" can be substituted for any of the open-ended linking verbs recited above, in order to change the scope of a given claim from what it would otherwise be using the open-ended linking verb.

Further, a device or system that is configured in a certain way is configured in at least that way, but it can also be configured in other ways than those specifically described.

The feature or features of one embodiment may be applied to other embodiments, even though not described or illustrated, unless expressly prohibited by this disclosure or the nature of the embodiments.

Some details associated with the embodiments described above and others are described below.

### BRIEF DESCRIPTION OF THE DRAWINGS

The following drawings illustrate by way of example and not limitation. For the sake of brevity and clarity, every feature of a given structure is not always labeled in every figure in which that structure appears. Identical reference numbers do not necessarily indicate an identical structure. Rather, the same reference number may be used to indicate a similar feature or a feature with similar functionality, as may non-identical reference numbers. Views in the figures are drawn to scale, unless otherwise noted, meaning the sizes of the depicted elements are accurate relative to each other for at least the embodiment in the view.
**FIG. 1A** is a perspective view of an embodiment of the present absorbent articles which is an incontinence guard and includes a longitudinally-extending recess configured to receive a phallus of a wearer.
**FIG. 1B** is a top view of the article of FIG. 1A.
**FIG. 1C** is a cross-sectional view of the article of FIG. 1A taken along line 1C-1C.
**FIG. 1D** is a bottom view of the article of FIG. 1A.
**FIG. 2A** is a perspective view of the article of FIG. 1A when the article is worn under an undergarment of a wearer.
**FIGs. 2B** and **2C** are cross-sectional views of the article of FIG. 1A, taken along line 2B-2B of FIG. 1D, when the article is in a state configured conform about the wearer's groin area and perineum; FIGs. 2B and 2C each illustrate the article with a portion of male anatomy.
**FIG. 3** is a top view of a second embodiment of the present absorbent articles which is an incontinence guard and includes a retaining member extending over at least a portion of a recess for stabilizing a phallus received therein.
**FIG. 4A** is a top view of a third embodiment of the present absorbent articles which is an adult incontinence brief in an open configuration.
**FIG. 4B** is a perspective view of the brief of FIG. 4A when the brief is worn by a wearer in a closed configuration.
**FIG. 5** is a cross-sectional view illustrating a structural configuration of some of the present absorbent articles including a laminate, an acquisition-distribution layer, a fluff core, and a backsheet.
**FIG. 6** is a cross-sectional view illustrating a structural configuration of some of the present absorbent articles including an unfolded laminate, a folded laminate, a fluff core, and a backsheet, where there are more folded layers within each longitudinal edge portion than within a recessed region disposed therebetween.
**FIG. 7** is a cross-sectional view illustrating a structural configuration of some of the present absorbent articles including multiple layers of unfolded laminates, a fluff core, and a backsheet, where there are more layers of unfolded laminates within each longitudinal edge portion than within a recessed region disposed therebetween.

### DETAILED DESCRIPTION OF ILLUSTRATIVE EMBODIMENTS

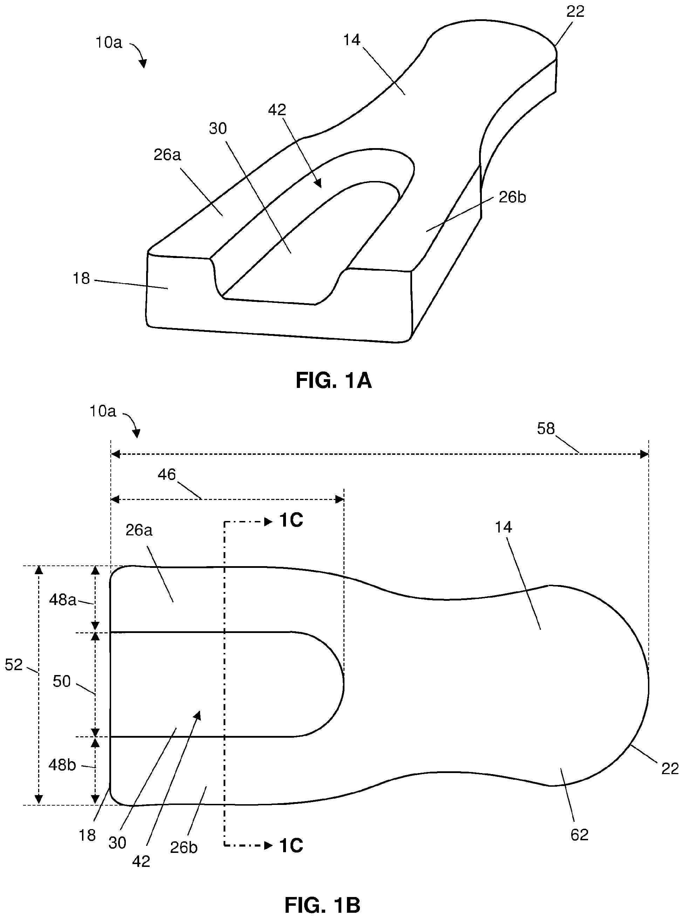

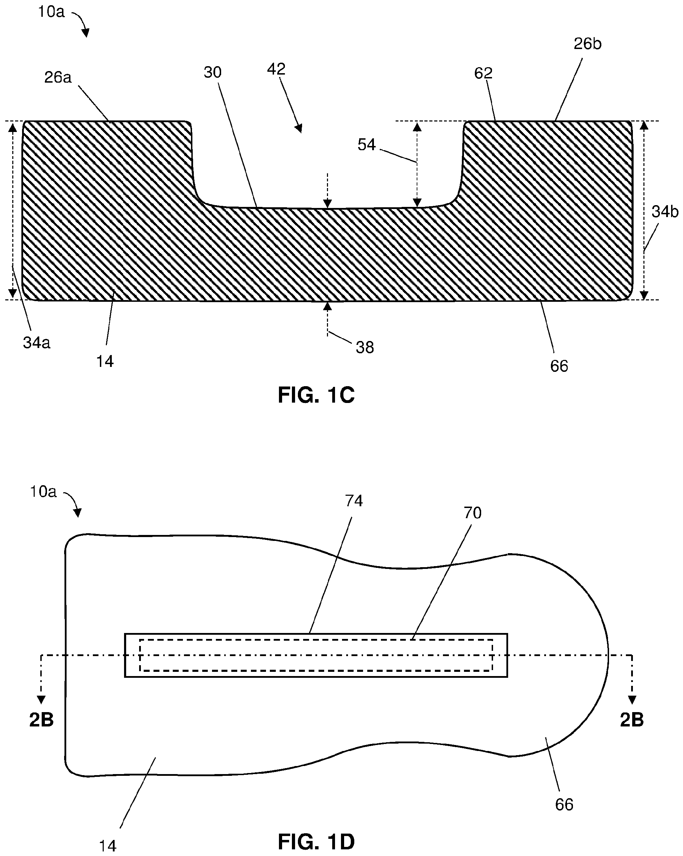

Referring to FIGs. 1A-1D, shown is an embodiment 10a of the present absorbent articles. Article 10a has a chassis 14 and, as shown, is an incontinence guard or pad (e.g., a bladder control pad). One or more portions of chassis 14 can comprise an absorbent material having superabsorbent polymer (SAP), cellulosic fibers, and/or other materials configured to absorb liquid such as urine. Examples of absorbent materials suitable for the present absorbent articles are described in further detail below. When worn, article 10a can be coupled to the inside of a clothing article, such as an undergarment or pants, with inner surface 62 facing the wearer and outer surface 66 facing away from the wearer and towards the clothing article. Adhesive 70, in the form of one or more adhesive portions and/or strips, can be disposed on outer surface 66 and can adhere to the wearer's clothing article to facilitate retention of article 10a. One or more liners 74 can be disposed on adhesive 70, and are configured to be removed before the wearer uses article 10a. Liner(s) 74 preserve the adhesiveness of adhesive 70 and prevent unintended adhesion to other objects. While article 10a, as shown, includes adhesive 70, some embodiments do not include the adhesive.

Chassis 14 has a length 58 extending longitudinally between a front edge portion 18 and a rear edge portion 22. A recessed region 30 of chassis 14 is disposed between longitudinal edge portions 26a and 26b. Chassis 14 can have different thicknesses within longitudinal edge portion 26a, longitudinal edge portion 26b, and recessed region 30. As shown in FIG. 1C, within longitudinal edge portions 26a and 26b, chassis 14 has thicknesses 34a and 34b, respectively, and within recessed region 30 the chassis has a thickness 38. Thickness 38 is at least 10% smaller than each of thicknesses 34a and 34b, such as for example, at least 10%, 20%, 30%, 40%, 50%, 60%, 70%, 80%, or 90% smaller than each of thicknesses 34a and 34b. Chassis 14 thereby defines a longitudinally-extending recess 42.

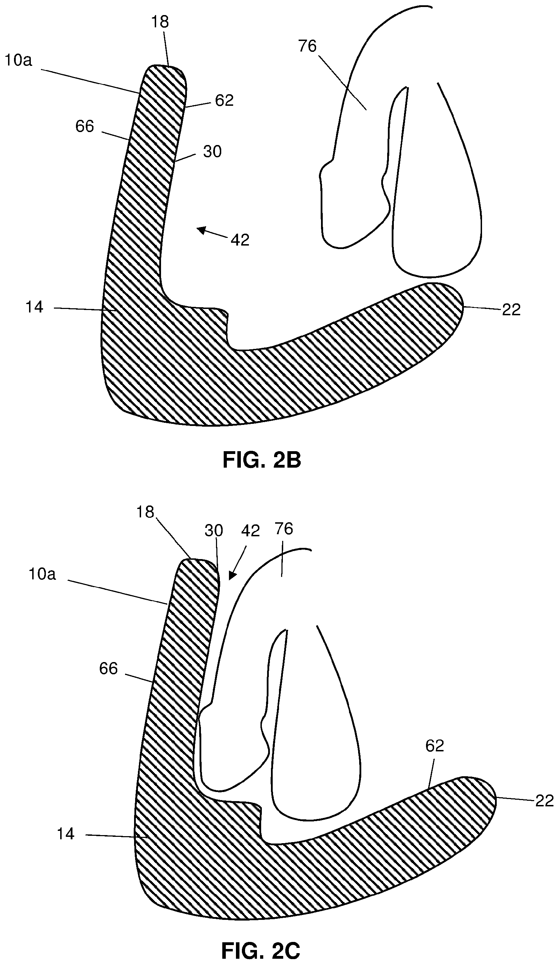

Recess 42 is sized and positioned to receive a phallus 76 of a wearer when article 10a is worn (FIGs. 2B-2C, described below). Recess 42 has a length 46 that can be at least 20% of length 58 of chassis 14. Length 46 can be, for example, greater than or equal to or between any two of 20%, 30%, 40%, 50%, 60%, 70%, 80%, 90%, or more (e.g., one-third) of length 58. When measured, length 46 can be greater than or equal to, or between any two of, 5, 6, 7, 8, 9, 10, 11, 12, or more (e.g., 5 or 6) centimeters. Recess 42 can, but need not, extend along the entire length of chassis 14 (e.g., length 46 can be at least 10% shorter than length 58). When recess 42 does not extend along the entire length of chassis 14, a relatively larger portion of inner surface 62 can contact the wearer. Not to be bound by any particular theory, the increased contact area promotes comfort by distributing the forces article 10a exerts on the wearer. The increased contact area can also promote article 10a's ability to wick away liquids from the wearer's skin. And chassis 14 can comprise relatively more absorbent material when recess 42 does not extend along the entire length of the chassis because the recess occupies less volume. The additional absorbent material can increase the absorption capacity of article 10a.

Recess 42 likewise can have a suitable width 50 and depth 54 for accommodating a phallus 76. Width 50 can be, for example, greater than or equal to or between any two of 10%, 20%, 30%, 40%, 50%, 60%, 70%, 80%, or 90% of width 52 of article 10a (e.g., at least 40% or at least 60% of width 52). To illustrate, width 50 can be, for example, greater than or equal to, or between any two of, 2, 3, 4, 5, 6, 7, 8, 9, 10, 11, 12, or more (e.g., 3 or 5) centimeters. And depth 54 can be, for example, greater than or equal to, or between any two of, 10%, 20%, 30%, 40%, 50%, 60%, 70%, 80%, 90%, or more of at least one of thicknesses 34a and 34b. To illustrate, depth 54 can be, for example, 1, 2, 3, 4, 5, 6, 7, or more (e.g., 1.5 or 3) centimeters. While the dimensions of recess 42 can be constant (e.g., the recess can be a rectangular prism), in some embodiments the recess has a variable width, length, and/or depth.

Each of edge portions 26a and 26b can be sized to facilitate appropriate sizing of recess 42 and such that the portion of inner surface 62 within the edge region has adequate surface area to promote comfort. For example, each of edge portions 26a and 26b can have a width (e.g., 48a and 48b, respectively) that is greater than or equal to, or between any two of, 5%, 10%, 15%, 20%, 25%, 30%, 35%, 40%, 45%, or more of width 52 (e.g., at least 5%, at least 20%, or at least 30% of width 52). As a result, a portion of inner surface 62 that is within each of edge regions 26a and 26b can be disposed above the bottom of recess 42 (e.g., by a distance less than, greater than, or equal to depth 54) and span a lateral distance less than or equal to the width of the edge region (e.g., 48a or 48b). As shown, for example, a portion of inner surface 62 within each of edge regions 26a and 26b is disposed above the bottom of recess 42 by a distance substantially equal to (e.g., within 10% of) depth 54 and spans a lateral distance substantially equal to (e.g., within 10% of) the width of the edge region (e.g., 48a and 48b). In other embodiments, however, each of edge regions 26a and 26b can have a variable thickness such that one or more portions of inner surface 62 within the edge region are disposed above the bottom of recess 42 by a distance smaller than (e.g., at least 10% smaller than) or greater than (e.g., at least 10% greater than) depth 54 and/or span a lateral distance smaller than (e.g., at least 10% smaller than) the thickness of the edge region (e.g., 48a or 48b).

Recess 42 can be positioned closer to front edge portion 18 than to rear edge portion 22. For example, as shown, recess 42 is defined through front edge portion 18. In other embodiments, however, recess 42 can be set back from front edge portion 18 such that recess 42 does not extend to the front of article 10a. Positioning recess 42 closer to front edge portion 18 than to rear edge portion 22 facilitates reception of a phallus 76 into the recess when article 10a is worn.

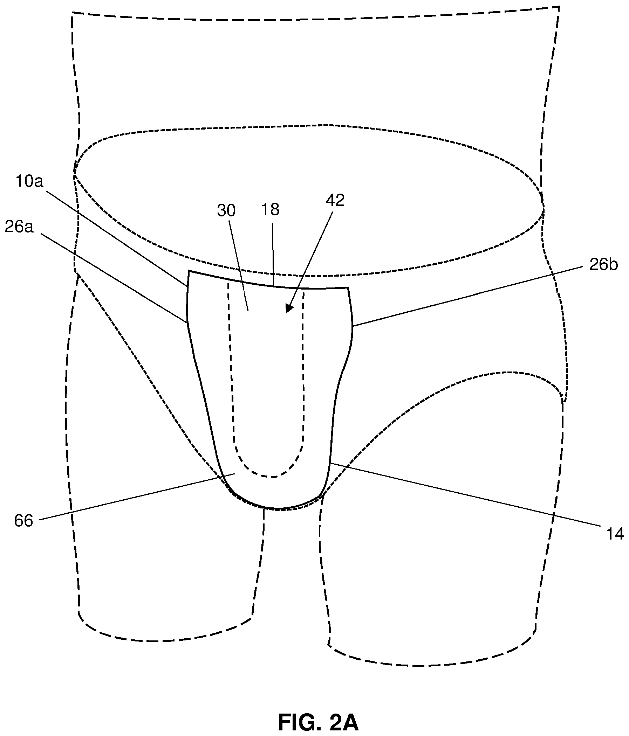

FIGs. 2A-2C illustrate use of article 10a (for clarity, adhesive 70 is not shown in FIGs. 2A-2C). As shown, article 10a is manipulated to conform at least about a wearer's groin area and/or perineum. Due at least in part to the above-described thickness variations in chassis 14 (e.g., thickness 38 is at least 10% smaller than each of thicknesses 34a and 34b), recess 42 remains defined both with (FIG. 2C) and without (FIG. 2B) a phallus 76 received therein, and both when the article is in a relaxed (e.g., flat) state (FIGs. 1A-1D) and a conformed state (FIGs. 2A-2C). Article 10a can thus accommodate phallus 76, which extends outwardly from the wearer's body, without substantial, if any, outward stretching and/or expansion of chassis 14 within recessed region 30. This configuration provides a number of advantages. Recess 42 promotes comfort at least by providing a space for phallus 76 to decrease the pressure exerted on the phallus by article 10a. Liquid can be received and contained in recess 42 until absorbed into chassis 14 to prevent leakage. Accommodation of phallus 76 within recess 42 can also manage leakage by mitigating the phallus' tendency to urge articles away from the wearer. Additionally, recess 42 can stabilize phallus 76 and, optionally, a dermal adhesive can be disposed on chassis 14 within recessed region 30 to further promote stabilization of the phallus.

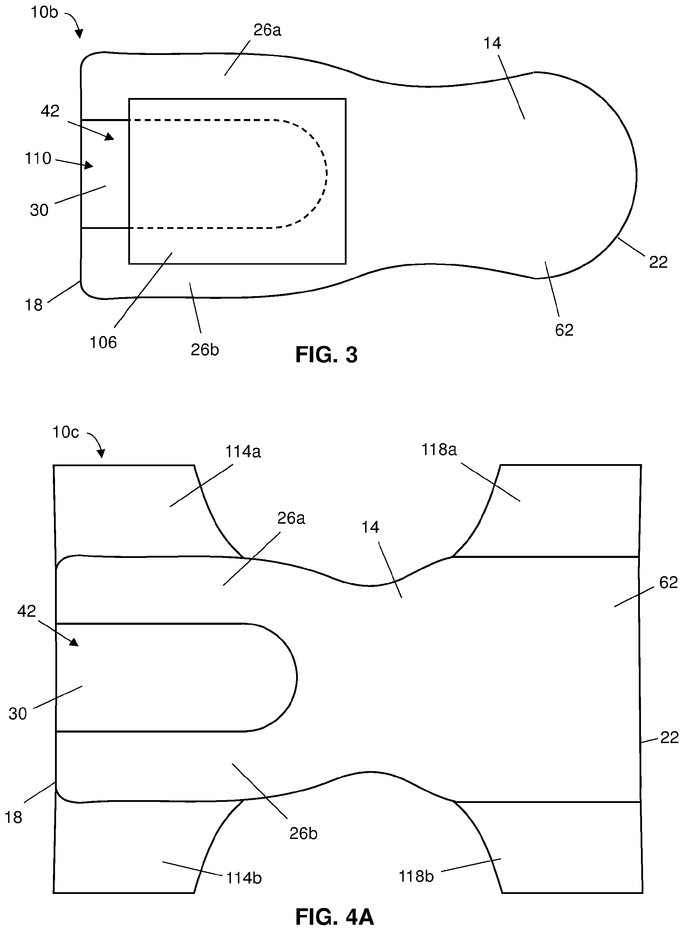

FIG. 3 shows another embodiment 10b of the present absorbent articles. Article 10b can be substantially similar to article 10a, the primary difference being that article 10b includes a retaining member 106 coupled to inner surface 62 of chassis 14. Retaining member 106 can extend between longitudinal edge portions 26a and 26b such that the retaining member covers at least a portion of recess 42. At least a portion of recess 42 can remain uncovered such that the recess and retaining member 106 together define an opening 110 configured to receive phallus 76. Retaining member 106 can be positioned such that opening 110 is disposed closer to front edge portion 18 than is the retaining member. Positioning retaining member 106 in this manner can promote easy entry of phallus 76 into recess 42. Retaining member 106 can promote stabilization of phallus 76 when the phallus is received in recess 42.

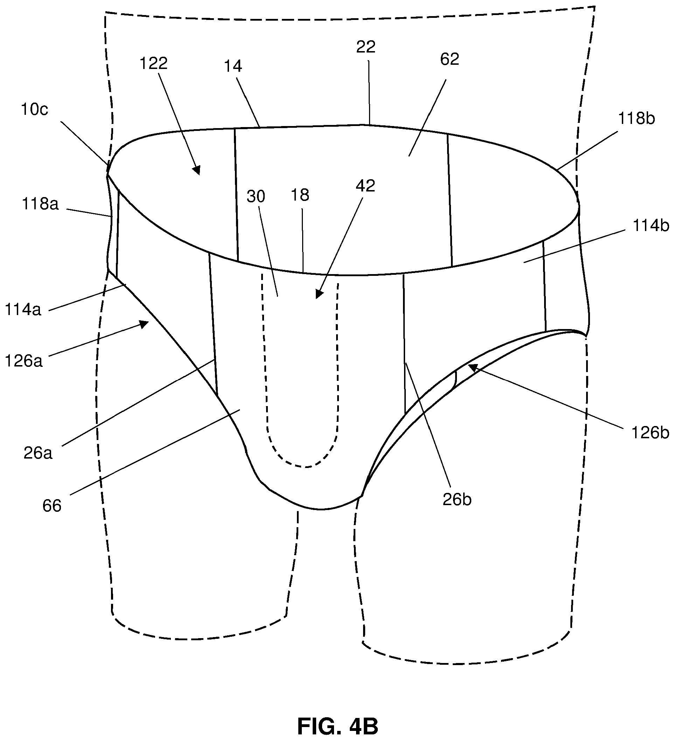

While each of articles 10a and 10b can be an incontinence guard or pad, other embodiments of the present absorbent articles can be any suitable absorbent article such as, for example, a baby diaper, training pant, adult incontinence brief or underwear, incontinence guard or pad, or the like. Such embodiments can have a chassis (e.g., 14) substantially similar to that of each of articles 10a and 10b. To illustrate, FIGs. 4A and 4B show an embodiment 10c of the present absorbent articles. Article 10c can be substantially similar to article 10a, the primary difference being that article 10c is an adult incontinence brief. Brief 10c can comprise a pair of front side panels 114a and 114b connected to chassis 14 at front edge portion 18 and a pair of rear side panels 118a and 118b connected to the chassis at rear edge portion 22. Brief 10c can be in an open configuration (FIG. 4A) and a closed configuration (FIG. 4B) in which the brief is worn. When brief 10c is in the closed configuration, front edge portion 18 can be coupled to rear edge portion 22 via front and rear side panels 114a, 114b, 118a, and 118b. For example, front side panels 114a and 114b can be coupled to rear side panels 118a and 118b, respectively, such that the side panels and chassis 14 together define a waist opening 122 and first and second leg openings 126a and 126b. While brief 10c is described with reference to adult incontinence briefs, in some embodiments the article can be a baby diaper or training pant.

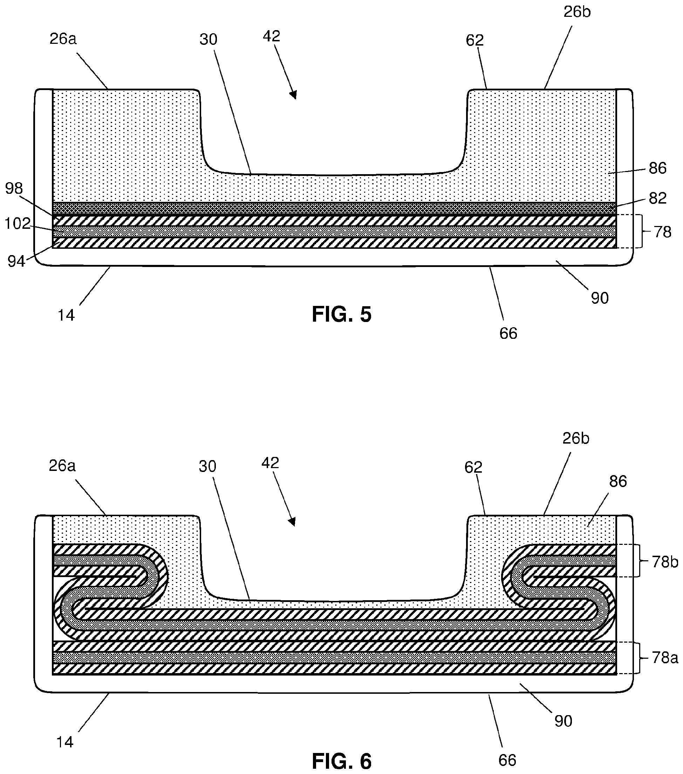

As described above, the present absorbent articles can comprise absorbent material. Absorbent materials suitable for use with the present absorbent articles include laminates, fluff, SAP, absorbent nonwoven substrates, and/or the like. A recessed region (e.g., 30) and/or longitudinal edge portions (e.g., 26a and 26b) can include absorbent material to absorb liquid received in a recess (e.g., 42). A chassis (e.g., 14) can incorporate the absorbent material in a configuration that facilitates formation of the recess. Each of FIGs. 5-7 illustrate a structural configuration suitable for embodiments of the present absorbent articles (e.g., 10a, 10b, 10c) to achieve such as recess.

In the configuration shown in FIG. 5, chassis 14 can comprise, within longitudinal edge portion 26a, longitudinal edge portion 26b, and recessed region 30, an acquisition-distribution layer (ADL) 82 layered between a laminate 78 and a fluff core 86. Fluff core 86 can be shaped to define recess 42, and can be layered above laminate 78 and ADL 82 such that, when the article is worn, the fluff core is closer to the wearer than are the laminate and ADL. Chassis 14 can, but need not, include a backsheet 90 at outer surface 66 and/or along at least a portion of the depthwise edges of the chassis.

ADL 82 can comprise, for example, a through-air bonded polymer nonwoven and can facilitate the distribution of liquid beyond the insult point thereof. ADL 82 can thus promote liquid distribution throughout fluff core 86 and laminate 78. Fluff core 86 and laminate 78 can absorb and retain liquid received in recess 42. For example, fluff core 86 can comprise cellulosic fibers and can have SAP dispersed throughout the fluff to promote absorbency. Laminate 78 can comprise an inner lamina 102 disposed between first outer lamina 94 and second outer lamina 98. Inner lamina 102 can comprise SAP, first outer lamina 94 can comprise tissue, and second outer lamina 98 can comprise at least one of a tissue and a nonwoven (e.g., a carded nonwoven). By way of illustration, laminate 78 can be formed by laying a mixture of SAP and adhesive-which becomes inner lamina 102-on first outer lamina 94, and thereafter layering second outer lamina 98 on top of the SAP-adhesive mixture.

While laminate 78, as shown, can have one inner lamina 102 comprising SAP (hereinafter, "SAP lamina") disposed between a first outer lamina 94 comprising tissue and a second outer lamina 98 comprising at least one of a tissue and a nonwoven (hereinafter, "substrate laminae"), in other embodiments the laminate can have any suitable number of SAP laminae and substrate laminae. Laminate 78 can comprise, for example, 1, 2, 3, 4, 5, 6, 7, 8, or more SAP laminae 102 and 1,2, 3, 4, 5, 6, 7, 8, or more substrate laminae 94 and/or 98 layered in any suitable order.

"Superabsorbent" or "superabsorbent material" or "SAP" refers to a water-swellable, water-insoluble organic or inorganic material capable, under the most favorable conditions, of absorbing at least about 15 times its weight in an aqueous solution containing 0.9 weight percent sodium chloride and, more desirably, at least about 30 times its weight in an aqueous solution containing 0.9 weight percent sodium chloride and, even more desirably, at least about 50 times its weight in an aqueous solution containing 0.9 weight percent sodium chloride. The SAP materials can be natural, synthetic and modified natural polymers and materials. In addition, the SAP materials can be inorganic materials, such as silica gels, or organic compounds such as cross linked polymers. SAPs that are suitable for at least some embodiments of the present absorbent articles are available from Sumitomo Seika Europe S.A. /N.V. in Belgium and/or from NA Industries, Inc. in Houston, Texas, USA. For example, in some embodiments, the SAP can have a centrifuge retention capacity of 20-60 grams per gram (g/g), for example 30-50 g/g, and/or a particle size distribution (PSD) with most or substantially all particles having a size between 150 µm and 850 µm. In some embodiments, the SAP can have a centrifuge retention capacity between 32 and 37 g/g, or alternatively between 44 and 48 g/g.

Backsheet 90 can be liquid-impermeable to prevent leakage into the wearer's clothing article(s). For example, backsheet 90 can comprise an inner liquid-impermeable film and an outer nonwoven backsheet that can be a nonwoven fabric. A "film" is a membrane-like layer of material formed of one or more polymers, which does not have a form consisting predominately of a web-like structure of fibers and/or other fibers. In some embodiments, backsheet 90 can be breathable, for example, an inner liquid-impermeable film of the backsheet can comprise a breathable film. The terms "breathable," "breathable film," "breathable laminate" or "breathable outer cover material" or "breathable backsheet" refers to a film, laminate, or outer cover material having a water vapor transmission rate ("WVTR") of at least about 300 grams/m²/24 hours. Breathable materials typically rely on molecular diffusion of vapor, and are substantially liquid impermeable. "Nonwoven" fabrics, according to an INDA definition, are broadly defined as sheet or web structures bonded together by entangling fiber or filaments (and by perforating films) mechanically, thermally, or chemically. They are flat, porous sheets that are made directly from separate fibers or from molten plastic or plastic film. They are not made by weaving or knitting and do not require converting the fibers to yarn. The basis weight of nonwoven fabrics is usually expressed as gsm or grams per square meter. "Nonwoven backsheet" is a backing substrate layer in the outer cover; a nonwoven backsheet is most often a nonwoven layer facing away from the wearer.

In the configuration shown in FIG. 6, chassis 14 can comprise a laminate 78b substantially similar to laminate 78 of FIG. 5. Laminate 78b can be folded and thereby layered on itself (hereinafter, "folded layers"). As shown, laminate 78b is folded such that there are three folded layers within each longitudinal edge portion 26a and 26b, and a single folded layer within recessed region 30 (e.g., laminate 78b is not layered on itself within the recessed region). Nevertheless, in other embodiments, laminate 78b can be folded in any suitable manner to provide an appropriate number of folded layers within longitudinal edge portion 26a, longitudinal edge portion 26b, and recessed region 30. For example, laminate 78b can be folded such that there are two or more folded layers in each of longitudinal edge portions 26a and 26b-such as, for example, greater than or equal to, or between any two of, 2, 3, 4, 5, 6, 7, 8, 9, 10, 11, 12, or more folded layers-and one or more folded layers in recessed region 30-such as, for example, greater than or equal to, or between any two of, 1, 2, 3, 4, 5, 6, 7, 8, 9, 10, 11, or more folded layers. There can be at least one more folded layer in each of longitudinal edge portions 26a and 26b than in recessed region 30 such that each of the longitudinal edge portions is thicker than the recessed region. The folded layers of laminate 78b can thereby define recess 42, or at least facilitate the definition thereof.

Laminate 78b can be layered on one or more unfolded laminates 78a, such as, for example, greater than or equal to, or between any two of, 1, 2, 3, 4, 5, 6, 7, 8, 9, 10, 11, or more unfolded laminates. Laminate(s) 78a can also be substantially similar to laminate 78 of FIG. 5. Providing additional layers of laminate(s)-whether those layers are folded layer(s) or additional unfolded laminate(s)-or laminate(s) having more SAP laminae and/or substrate laminae can improve the absorption capacity of the article. A fluff core 86, which is substantially similar to that of FIG. 5, can be layered on laminate 78b to further improve absorption. Fluff core 86 and folded laminate 78b can together facilitate the definition of recess 42. Chassis 14 can optionally include a backsheet 90 substantially similar to that of FIG. 5.

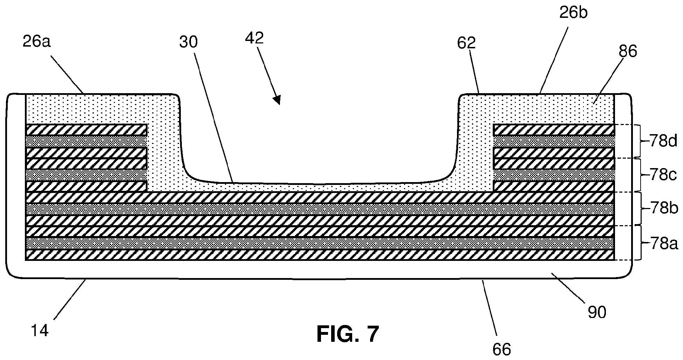

FIG. 7 illustrates a structural configuration substantially similar to that shown in FIG. 6, except that chassis 14 can have at least one more layer of unfolded laminates (e.g., 78c and 78d) in each of longitudinal edge portions 26a and 26b than in recessed region 30.

The structural configurations illustrated in FIGs. 5-7 are provided by way of example, and not by way of limitation. The present absorbent articles can comprise or omit any of the various elements described in connection with FIGs. 5-7, such as, for example, fluff core(s), unfolded laminate(s), folded laminate(s), ADL(s), backsheet(s), absorbent material(s) and/or the like. For example, some articles can comprise an ADL (e.g., 82) disposed between a folded laminate (e.g., 78a) and an unfolded laminate (e.g., 78b). By way of further example, some articles can include a liquid-permeable topsheet configured to be closest to the user during use (e.g., disposed at the inner surface (e.g., 62)). And while FIGs. 5-7 each show configurations having a fluff core (e.g., 86), some articles do not have a fluff core. Some articles, for example, comprise a folded laminate-without a fluff core and/or without unfolded laminate-that is configured to be closest to the wearer during use.

Some embodiments of the present methods for making an absorbent article (e.g., 10a, 10b, 10c) comprise folding a laminate (e.g., 78b) to form a chassis (e.g., 14). The laminate can be folded such that two or more folded layers of the laminate are within each longitudinal edge portion (e.g., 26a and 26b) of the chassis, and one or more folded layers of the laminate are within a recessed region (e.g., 30) of the chassis. Folding can be performed such that each of the longitudinal edge portions has at least one more folded layer than the recessed region. A thickness of the chassis within the recessed region (e.g., 38) can thereby be at least 10% smaller than a thickness of the chassis within each of the longitudinal edge portions (e.g., 34a, 34b) to define a longitudinally-extending recess (e.g., 42). The laminate can be any suitable laminate, such as any of those described above. In some methods, forming the chassis can further comprise incorporating the folded laminate with other elements. For example, some methods comprise layering the folded laminate with one or more other absorbent materials, fluff core(s) (e.g., 86), ADL(s), (e.g., 82), unfolded laminate(s) (e.g., 78, 78a, 78c-78d), backsheet(s) (e.g., 90), topsheet(s), and/or the like. Layering can be performed to achieve any suitable structural configuration, such as any of the configurations described above.

The above specification and examples provide a complete description of the structure and use of illustrative embodiments. Although certain embodiments have been described above with a certain degree of particularity, or with reference to one or more individual embodiments, those skilled in the art could make numerous alterations to the disclosed embodiments without departing from the scope of this invention. As such, the various illustrative embodiments of the methods and systems are not intended to be limited to the particular forms disclosed. Rather, they include all modifications and alternatives falling within the scope of the claims, and embodiments other than the one shown may include some or all of the features of the depicted embodiment. For example, elements may be omitted or combined as a unitary structure, and/or connections may be substituted. Further, where appropriate, aspects of any of the examples described above may be combined with aspects of any of the other examples described to form further examples having comparable or different properties and/or functions, and addressing the same or different problems. Similarly, it will be understood that the benefits and advantages described above may relate to one embodiment or may relate to several embodiments.

The claims are not intended to include, and should not be interpreted to include, means-plus- or step-plus-function limitations, unless such a limitation is explicitly recited in a given claim using the phrase(s) "means for" or "step for," respectively.

## Claims

1. An absorbent article (10a, 10b, 10c) comprising:
a chassis (14) having:
a length (58) extending longitudinally between opposing front and rear edge portions (18, 22); and
a recessed region (30) disposed between opposing first and second
longitudinal edge portions (26a, 26b), the chassis (14) comprising an absorbent material within the recessed region (30);
wherein a thickness (38) of the chassis (14) within the recessed region (30) is at least 10% smaller than a thickness (34a, 34b) of the chassis (14) within each of the first and second longitudinal edge portions (26a, 26b) such that the chassis (14) defines a longitudinally-extending recess (42) configured to receive a phallus of a wearer,
**characterized in that**
- the absorbent material comprises a fluff core (86),
- the chassis (14) comprises, within the longitudinal front and rear edge portions (26a, 26b) and the recessed region (30) an acquisition- distribution layer (82) layered between a laminate (78) and the fluff core (86),
- the fluff core (86) is shaped to define the recess (42) and is layered above the laminate (78) and the acquisition- distribution layer (82) such that, when the article is worn, the fluff core is closer to the wearer than are the laminate and the acquisition- distribution layer (82).

2. The absorbent article (10a, 10b, 10c) of claim 1, wherein:
the absorbent material comprises a laminate (78, 78a-78d) having an inner lamina (102) disposed between first and second outer laminae (94, 98); and
the inner lamina (102) comprises superabsorbent polymer, the first outer lamina (94) comprises tissue, and the second outer lamina (98) comprises at least one of a tissue and a carded nonwoven.

3. The absorbent article (10a, 10b, 10c) of claim 1, wherein the absorbent material comprises a through-air bonded polymer nonwoven disposed between the laminate (78, 78a-78d) and the core (86).

4. The absorbent article (10a, 10b, 10c) of any of claims 2-3, wherein the laminate (78, 78a-78d) is folded such that the chassis (14) comprises:
one or more folded layers of the laminate (78, 78a-78d) within the recessed region (30); and
for each of the first and second longitudinal edge portions (26a, 26b), two or more folded layers of the laminate (78, 78a-78d) such that there are more folded layers within the longitudinal edge portion than within the recessed region (30).

5. The absorbent article (10a, 10b, 10c) of any of claims 1-4, wherein the recess (42) extends a longitudinal distance (46) that is at least one-third of the length (58) of the chassis (14).

6. The absorbent article (10a, 10b, 10c) of any of claims 1-5, wherein the recess (42) has:
a length (46), measured along a longitudinal direction, that is at least 5 cm;
a width (50), measured along a latitudinal direction extending between the first and second longitudinal edges, that is at least 3 centimeters (cm); and
a depth (54), measured along a direction perpendicular to the latitudinal and longitudinal directions, that is at least 1.5 cm.

7. The absorbent article (10a, 10b, 10c) of any of claims 1-6, wherein the recess (42) is disposed closer to the front edge portion (18) than to the rear edge portion (22).

8. The absorbent article (10a, 10b, 10c) of claim 7, wherein the recess (42) extends from the front edge portion (18) along a longitudinal distance that is at least 10% shorter than the length (58) of the chassis (14).

9. The absorbent article (10a, 10b, 10c) of any of claims 1-8, comprising a retaining member (106) that extends between the first and second longitudinal edge portions (26a, 26b) over at least a portion of the recess (42) such that the retaining member (106) and the recess (42) together define a chamber and an opening (110), the opening (110) configured to receive a phallus of a wearer into the chamber.

10. The absorbent article (10a, 10b, 10c) of claim 9, wherein the opening (110) is disposed closer to the front edge portion (18) than is the retaining member (106).

11. The absorbent article (10a, 10b, 10c) of any of claims 1-10, wherein the absorbent article (10a, 10b, 10c) is an incontinence guard.

12. The absorbent article (10a, 10b, 10c) of claim 11, wherein the chassis (14) haps:
opposing inner and outer surfaces (62, 66); and
an adhesive (70) disposed on the outer surface (66);
wherein, when the article is worn by a wearer, the inner surface (62) faces the wearer and the adhesive (70) is configured to adhere to a clothing article of the wearer.

13. The absorbent article (10a, 10b, 10c) of any of claims 1-10, wherein a first side panel (114a, 114b) of the front edge portion (18) is configured to be coupled to a first side panel (118a) of the rear edge portion (22) and a second side panel (114a, 114b) of the front edge portion (18) is configured to be coupled to a second side panel (118a, 118b) of the rear edge portion (22) to define a closed configuration in which the front and rear edge portions (18, 22) cooperate to encircle and define a waist opening (122), a left side of the chassis (14) defines a first leg opening (126a), and a right side of the chassis (14) defines a second leg opening (126b).

14. A method of making an absorbent article (10a, 10b, 10c) as defined in claim 1, the method comprising:
folding a laminate (78, 78a-78d) to form at least a part of a chassis (14) such that the chassis (14) has:
two or more folded layers of the laminate (78, 78a-78d) within each of a first longitudinal edge portion (26a, 26b) and a second longitudinal edge portion (26b, 26b); and
one or more folded layers of the laminate (78, 78a-78d) within a recessed region (30) disposed between the first and second longitudinal edge portions (26a, 26b);
wherein each of the first and second longitudinal edge portions (26a, 26b) has more folded layers than the recessed region (30) such that a thickness (38) of the chassis (14) within the recessed region (30) is at least 10% smaller than a thickness (34a, 34b) of the chassis (14) within each of the first and second longitudinal edge portions (26a, 26b), the chassis (14) thereby defining a longitudinally-extending recess (42) configured to receive a phallus of a wearer;
wherein the laminate (78, 78a-78d) has an inner lamina (102) disposed between first and second outer laminae (94, 98), the inner lamina (102) comprising superabsorbent polymer, the first outer lamina (94) comprising tissue, and the second outer lamina (98) comprising at least one of a tissue and a nonwoven,
**characterized in that**
- an acquisition- distribution layer (82) is layered between a laminate (78) and a fluff core (86), thereby adding the acquisition- distribution layer (82) to the chassis (14) within the first and second longitudinal edge portions (26a, 26b), and the recessed region (30); and **in that**
- the method further comprises shaping the fluff core (86) to define the recess (42) and layering the fluff core (86) above the laminate (78) and the acquisition- distribution layer (82) such that, when the article is worn, the fluff core is closer to the wearer than are the laminate and the acquisition-distribution layer (82).

## Patentansprüche

1. Ein absorbierender Artikel (10a 10b, 10c), aufweisend
ein Chassis (14) mit
einer Länge (58), die sich in Längsrichtung zwischen gegenüberliegenden Vorder- und Hinterrandabschnitten (18, 22) erstreckt; und
einen ausgesparten Bereich (30), der zwischen gegenüberliegenden ersten und zweiten Längsrandabschnitten (26a, 26b) angeordnet ist, wobei das Chassis (14) ein absorbierendes Material innerhalb des vertieften Bereichs (30) aufweist;
wobei eine Dicke (38) des Chassis (14) innerhalb des ausgesparten Bereichs (30) mindestens 10 % kleiner ist als eine Dicke (34a, 34b) des Chassis (14) innerhalb jedes der ersten und zweiten Längsrandabschnitte (26a, 26b), sodass das Chassis (14) eine sich in Längsrichtung erstreckende Aussparung (42) definiert, die zur Aufnahme eines Phallus eines Trägers konfiguriert ist,
**dadurch gekennzeichnet, dass**
- der absorbierende Artikel einen Flockenzellstoffkern (86) aufweist,
- das Chassis (14) innerhalb der länglichen Vorder- und Hinterrandabschnitte (26a, 26b) und des ausgesparten Bereichs (30) eine Aufnahme-Verteilungs-Schicht (82) aufweist, zwischen einem Schichtstoff (78) und dem Flockenzellstoffkern (86) geschichtet ist,
- der Flockenzellstoffkern (86) geformt ist, um die Aussparung (42) zu definieren und über dem Schichtstoff (78) und der Aufnahme-Verteilungs-Schicht (82) geschichtet ist, so dass, wenn der Artikel getragen wird, der Flockenzellstoffkern näher am Träger ist als der Schichtstoff und die Aufnahme-Verteilungs-Schicht (82).

2. Der absorbierende Artikel (10a, 10b, 10c) nach Anspruch 1, wobei:
das absorbierende Material einen Schichtstoff (78, 78a-78d) aufweist mit einer inneren Lage (102), die zwischen ersten und zweiten äußeren Lagen (94, 98) angeordnet ist; und
die innere Lage (102) ein superabsorbierendes Polymer aufweist, die erste äußere Lage (94) Gewebe aufweist, und die zweite äußere Lage (98) mindestens eines von einem Gewebe und einem kardierten Vlies aufweist.

3. Der absorbierende Artikel (10a, 10b, 10c) nach Anspruch 1, wobei das absorbierende Material einen durch Luft gebundenen Polymervliesstoff (through-air bonded polymer nonwoven) aufweist, der zwischen dem Schichtstoff (78, 78a-78d) und dem Kern (86) angeordnet ist.

4. Der absorbierende Artikel (10a, 10b, 10c) nach einem der Ansprüche 2 - 3, wobei der Schichtstoff (78, 78a-78d) so gefaltet ist, dass das Chassis (14) Folgendes aufweist:
eine oder mehrere gefaltete Schichten des Schichtstoffs (78, 78a-78d) innerhalb des ausgesparten Bereichs (30); und
für jeden der ersten und zweiten Längsrandabschnitte (26a, 26b), zwei oder mehr gefaltete Schichten (78, 78a-78d) des Schichtstoffs, so dass es mehr gefaltete Schichten innerhalb des Längsrandabschnitts als innerhalb des ausgesparten Bereichs (30) gibt.

5. Der absorbierende Artikel (10a, 10b, 10c) nach einem der Ansprüche 1-4, wobei sich die Aussparung (42) über eine Längsstrecke (46) erstreckt, die mindestens ein Drittel (58) der Länge des Chassis (14) entspricht.

6. Der absorbierende Artikel (10a, 10b, 10c) nach einem der Ansprüche 1-5, wobei die Aussparung (42) Folgendes aufweist:
eine Länge (46), gemessen entlang einer Längsrichtung, die mindestens 5 cm beträgt;
eine Breite (50), gemessen entlang einer Breitenrichtung, die sich zwischen den ersten und zweiten Längsrändern erstreckt, die mindestens 3 Zentimeter (cm) beträgt; und
eine Tiefe (54), gemessen entlang einer Richtung senkrecht zur Breiten- und Längsrichtung, die mindestens 1,5 cm beträgt.

7. Der absorbierende Artikel (10a, 10b, 10c) nach einem der Ansprüche 1-6, wobei die Aussparung (42) näher zum Vorderrandabschnitt (18) als zum Hinterrandabschnitt (22) angeordnet ist.

8. Der absorbierende Artikel (10a, 10b, 10c) nach Anspruch 7, wobei die Aussparung (42) sich von dem Vorderrandabschnitt (18) entlang einer Längsstrecke erstreckt, die mindestens 10 % kürzer ist als die Länge (58) des Chassis (14).

9. Der absorbierende Artikel (10a, 10b, 10c) nach einem der Ansprüche 1-8, aufweisend ein Halteelement (106), das sich zwischen den ersten und zweiten Längsrandabschnitten (26a, 26b) über mindestens einen Teil der Aussparung (42) erstreckt, sodass das Halteelement (106) und die Aussparung (42) zusammen eine Kammer und eine Öffnung (110) definieren, wobei die Öffnung (110) dazu konfiguriert ist, einen Phallus eines Trägers in die Kammer aufzunehmen.

10. Der absorbierende Artikel (10a, 10b, 10c) nach Anspruch 9, wobei die Öffnung (110) näher am Vorderrandabschnitt (18) angeordnet ist als das Halteelement (106).

11. Der absorbierende Artikel (10a, 10b, 10c) nach einem der Ansprüche 1-10, wobei der absorbierende Artikel (10a, 10b, 10c) ein Inkontinenzschutz ist.

12. Der absorbierende Artikel (10a, 10b, 10c) nach Anspruch 11, wobei das Chassis (14) Folgendes aufweist:
gegenüberliegende Innen- und Außenflächen (62, 66); und
einen auf der Außenfläche (66) angeordneten Klebstoff (70);
wobei, wenn der Artikel von einem Träger getragen wird, die Innenfläche (62) dem Träger zugewandt ist und der Klebstoff (70) so konfiguriert ist, dass er an einem Kleidungsstück des Trägers haftet.

13. Der absorbierende Artikel (10a, 10b, 10c) nach einem der Ansprüche 1-10, wobei ein erstes Seitenfeld (114a, 114b) des Vorderrandabschnitts (18) konfiguriert ist, um mit einem ersten Seitenfeld (118a) des Hinterrandabschnitts (22) verbunden zu werden, und ein zweites Seitenfeld (114a, 114b) des Vorderrandabschnitts (18) konfiguriert ist, um mit einem zweiten Seitenfeld (118a, 118b) des Hinterrandabschnitts (22) verbunden zu werden, um eine geschlossene Konfiguration zu definieren, in der die Vorder- und Hinterrandabschnitte (18, 22) zusammenwirken, um eine Taillenöffnung (122) zu umschließen und zu definieren, eine linke Seite des Chassis (14) eine erste Beinöffnung (126a) definiert und eine rechte Seite des Chassis (14) definiert eine zweite Beinöffnung (126b).

14. Ein Verfahren zur Herstellung eines absorbierenden Artikels (10a, 10b, 10c) wie in Anspruch 1 definiert, wobei das Verfahren Folgendes aufweist:
Falten eines Schichtstoffs (78, 78a-78d), um mindestens einen Teil eines Chassis (14) zu bilden, sodass das Chassis (14) Folgendes aufweist:
zwei oder mehr gefaltete Schichten des Schichtstoffs (78, 78a-78d), jeweils innerhalb eines ersten Längsrandabschnitts (26a, 26b) und eines zweiten Längsrandabschnitts (26a, 26b); und
eine oder mehrere gefaltete Schichten des Schichtstoffs (78, 78a-78d), innerhalb eines ausgesparten Bereichs (30), der zwischen den ersten und zweiten Längsrandabschnitten (26a, 26b) angeordnet ist;
wobei jeder der ersten und zweiten Längsrandabschnitte (26a, 26b) mehr gefaltete Schichten aufweist als der ausgesparte Bereich (30), so dass eine Dicke (38) des Chassis (14) innerhalb des ausgesparten Bereichs (30) mindestens 10 % kleiner ist als eine Dicke (34a, 34b) des Chassis (14) innerhalb jedes der ersten und zweiten Längsrandabschnitte (26a, 26b), wobei das Chassis (14) dadurch eine sich in Längsrichtung erstreckende Aussparung (42) definiert, die zur Aufnahme eines Phallus eines Trägers konfiguriert ist;
wobei der Schichtstoff (78, 78a-78d) eine innere Lage (102) aufweist, welche zwischen ersten und der zweiten äußeren Lagen (94, 98) angeordnet ist, wobei die innere Lage (102) superabsorbierendes Polymer aufweist, die erste äußere Lage (94) Gewebe aufweist, und die zweite äußere Lage (98) mindestens eines von einem Gewebe und einem Vlies aufweist,
**dadurch gekennzeichnet, dass**
- eine Aufnahme-Verteilungs-Schicht (82) zwischen einem Schichtstoff (78) und einem Flockenzellstoffkern (86) geschichtet ist, wodurch die Aufnahme-Verteilungs-Schicht (82) dem Chassis (14) innerhalb der ersten und zweiten Längsrandabschnitte (26a, 26b) und dem ausgesparten Bereich (30) hinzugefügt wird; und dass
- das Verfahren weiterhin Ausformen des Flockenzellstoffkerns (86) aufweist, um die Aussparung (42) zu definieren und Schichten des Flockenzellstoffkerns (86) über den Schichtstoff (78) und die Aufnahme-Verteilungs-Schicht (82), so dass, wenn der Artikel getragen wird, der Flockenzellstoffkern näher am Träger ist als der Schichtstoff und die Aufnahme-Verteilungs-Schicht (82).

## Revendications

1. Article absorbant (10a, 10b, 10c) comprenant :
un châssis (14) ayant :
une longueur (58) s'étendant longitudinalement entre des portions de bord avant et arrière (18, 22) opposées ; et
une région évidée (30) disposée entre des première et seconde portions de bord longitudinal (26a, 26b) opposées, le châssis (14) comprenant un matériau absorbant au sein de la région évidée (30) ;
dans lequel une épaisseur (38) du châssis (14) au sein de la région évidée (30) est au moins 10 % inférieure à une épaisseur (34a, 34b) du châssis (14) au sein de chacune des première et seconde portions de bord longitudinal (26a, 26b), de telle sorte que le châssis (14) définit un évidement (42) s'étendant longitudinalement configuré pour recevoir un phallus d'un porteur,
**caractérisé en ce que**
- le matériau absorbant comprend une âme en peluche (86),
- le châssis (14) comprend, au sein des portions de bord longitudinal avant et arrière (26a, 26b) et de la région évidée (30), une couche d'acquisition-distribution (82) superposée entre un stratifié (78) et l'âme en peluche (86),
- l'âme en peluche (86) est façonnée pour définir l'évidement (42) et est superposée au-dessus du stratifié (78) et de la couche d'acquisition-distribution (82), de telle sorte que, lorsque l'article est porté, l'âme en peluche est plus près du porteur que ne le sont le stratifié et la couche d'acquisition-distribution (82).

2. Article absorbant (10a, 10b, 10c) selon la revendication 1, dans lequel :
le matériau absorbant comprend un stratifié (78, 78a à 78d) comportant un feuillet interne (102) disposé entre des premier et second feuillets externes (94, 98) ; et
le feuillet interne (102) comprend un polymère superabsorbant, le premier feuillet externe (94) comprend un tissu, et le second feuillet externe (98) comprend au moins l'un d'un tissu et d'un non-tissé cardé.

3. Article absorbant (10a, 10b, 10c) selon la revendication 1, le matériau absorbant comprenant un non-tissé polymère lié à l'air disposé entre le stratifié (78, 78a à 78d) et l'âme (86).

4. Article absorbant (10a, 10b, 10c) selon l'une quelconque des revendications 2 et 3, dans lequel le stratifié (78, 78a à 78d) est plié de telle sorte que le châssis (14) comprend :
une ou plusieurs couches pliées du stratifié (78, 78a à 78d) au sein de la région évidée (30) ; et
pour chacune des première et seconde portions de bord longitudinal (26a, 26b), deux couches pliées ou plus du stratifié (78, 78a à 78d) de telle sorte qu'il y a plus de couches pliées au sein de la portion de bord longitudinal qu'au sein de la région évidée (30).

5. Article absorbant (10a, 10b, 10c) selon l'une quelconque des revendications 1 à 4, dans lequel l'évidement (42) s'étend sur une distance longitudinale (46) qui représente au moins un tiers de la longueur (58) du châssis (14).

6. Article absorbant (10a, 10b, 10c) selon l'une quelconque des revendications 1 à 5, dans lequel l'évidement (42) a :
une longueur (46), mesurée suivant une direction longitudinale, qui est d'au moins 5 cm ;
une largeur (50), mesurée suivant une direction latitudinale s'étendant entre les premier et second bords longitudinaux, qui est d'au moins 3 centimètres (cm) ; et
une profondeur (54), mesurée suivant une direction perpendiculaire aux directions latitudinale et longitudinale, qui est d'au moins 1,5 cm.

7. Article absorbant (10a, 10b, 10c) selon l'une quelconque des revendications 1 à 6, dans lequel l'évidement (42) est disposé plus près de la portion de bord avant (18) que de la portion de bord arrière (22).

8. Article absorbant (10a, 10b, 10c) selon la revendication 7, dans lequel l'évidement (42) s'étend à partir de la portion de bord avant (18) suivant une distance longitudinale qui est au moins 10 % plus courte que la longueur (58) du châssis (14).

9. Article absorbant (10a, 10b, 10c) selon l'une quelconque des revendications 1 à 8, comprenant un organe de retenue (106) qui s'étend entre les première et seconde portions de bord longitudinal (26a, 26b) sur au moins une portion de l'évidement (42) de telle sorte que l'organe de retenue (106) et l'évidement (42) définissent conjointement une chambre et une ouverture (110), l'ouverture (110) étant configurée pour recevoir un phallus d'un porteur dans la chambre.

10. Article absorbant (10a, 10b, 10c) selon la revendication 9, dans lequel l'ouverture (110) est disposée plus près de la portion de bord avant (18) que ne l'est l'organe de retenue (106).

11. Article absorbant (10a, 10b, 10c) selon l'une quelconque des revendications 1 à 10, dans lequel l'article absorbant (10a, 10b, 10c) est une protection contre l'incontinence.

12. Article absorbant (10a, 10b, 10c) selon la revendication 11, dans lequel le châssis (14) comporte :
des surfaces interne et externe opposées (62, 66) ; et
un adhésif (70) disposé sur la surface externe (66) ;
dans lequel, lorsque l'article est porté par un porteur, la surface interne (62) fait face au porteur et l'adhésif (70) est configuré pour adhérer à un article vestimentaire du porteur.

13. Article absorbant (10a, 10b, 10c) selon l'une quelconque des revendications 1 à 10, dans lequel un premier panneau latéral (114a, 114b) de la portion de bord avant (18) est configuré pour être couplé à un premier panneau latéral (118a) de la portion de bord arrière (22) et un second panneau latéral (114a, 114b) de la portion de bord avant (18) est configuré pour être couplé à un second panneau latéral (118a, 118b) de la portion de bord arrière (22) pour définir une configuration fermée dans laquelle les portions de bord avant et arrière (18, 22) coopèrent pour encercler et définir une ouverture de taille (122), un côté gauche du châssis (14) définit une première ouverture de jambe (126a), et un côté droit du châssis (14) définit une seconde ouverture de jambe (126b).

14. Procédé de fabrication d'un article absorbant (10a, 10b, 10c) tel que défini dans la revendication 1, le procédé comprenant :
le pliage d'un stratifié (78, 78a à 78d) pour former au moins une partie d'un châssis (14) de telle sorte que le châssis (14) comporte :
deux couches pliées ou plusieurs du stratifié (78, 78a à 78d) au sein de chacune d'une première portion de bord longitudinal (26a, 26b) et d'une seconde portion de bord longitudinal (26b, 26b) ; et
une ou plusieurs couches pliées du stratifié (78, 78a à 78d) au sein d'une région évidée (30) disposée entre les première et seconde portions de bord longitudinal (26a, 26b) ;
dans lequel chacune des première et seconde portions de bord longitudinal (26a, 26b) comporte plus de couches pliées que la région évidée (30), de telle sorte qu'une épaisseur (38) du châssis (14) au sein de la région évidée (30) est au moins 10 % inférieure à une épaisseur (34a, 34b) du châssis (14) au sein de chacune des première et seconde portions de bord longitudinal (26a, 26b), le châssis (14) définissant ainsi un évidement (42) s'étendant longitudinalement et configuré pour recevoir un phallus d'un porteur ;
dans lequel le stratifié (78, 78a à 78d) comporte un feuillet interne (102) disposé entre des premier et second feuillets externes (94, 98), le feuillet interne (102) comprenant un polymère superabsorbant, le premier feuillet externe (94) comprenant un tissu, et le second feuillet externe (98) comprenant au moins l'un d'un tissu et d'un non-tissé,
**caractérisé en ce que**
- une couche d'acquisition-distribution (82) est superposée entre un stratifié (78) et une âme en peluche (86), ajoutant ainsi la couche d'acquisition-distribution (82) au châssis (14) au sein des première et seconde portions de bord longitudinal (26a, 26b) et de la région d'évidement (30) ; et **en ce que**
- le procédé comprend en outre le façonnage de l'âme en peluche (86) pour définir l'évidement (42) et la superposition de l'âme en peluche (86) au-dessus du stratifié (78) et de la couche d'acquisition-distribution (82), de telle sorte que, lorsque l'article est porté, l'âme en peluche est plus près du porteur que ne le sont le stratifié et la couche d'acquisition-distribution (82).
